# FASCICULE DE BREVET EUROPEEN

(11) **EP 3 996 781 B1**
(45) Date de publication et mention de la délivrance du brevet: **06.09.2023**
(21) Numéro de dépôt: 20750705.4
(22) Date de dépôt: 09.07.2020
(51) Int. Cl.: A61M 11/02, A61M 15/00, A61M 15/08, B05B 11/06

(54) **DISPOSITIF DE DISTRIBUTION NASALE DE POUDRE**
VORRICHTUNG ZUR NASALEN PULVERVERABREICHUNG
NASAL POWDER DELIVERY DEVICE

(30) Priorité: 10.07.2019 FR 1907756
(43) Date de publication de la demande: 18.05.2022
(73) Titulaire: APTAR France SAS, 27110 Le Neubourg (FR)
(72) Inventeur: POULLAIN, Franck, 27400 La Haye Malherbe (FR); CAMPOT, Anne-Cécile, 27340 Terres de Bord (FR); CABILLIC, Gwénollé, 76710 Bosc Guerard Saint Adrien (FR); ARNETT, Jaime, Fishers, IN 46037 (US)
(74) Mandataire: CAPRI
(86) Numéro de dépôt international: PCT/FR2020/051232
(87) Numéro de publication internationale: WO 2021/005311

(56) Documents cités:
- WO-A1-2017/191400
- US-A1- 2016 296 957
- US-B1- 6 321 942
- US-B1- 6 708 846

## Description

La présente invention concerne un dispositif de distribution nasale de poudre.

Les dispositifs de distribution nasale de poudre sont bien connus. Ils comportent généralement un réservoir contenant une ou plusieurs doses de poudre, des moyens de distribution, et une tête de distribution nasale destinée à être insérée dans une narine d'un utilisateur, ladite tête de distribution nasale comportant un orifice de distribution. Les moyens de distribution comportent généralement une chasse d'air. Lorsque le dispositif de distribution est actionné, une dose de poudre est distribuée dans une narine de l'utilisateur.

Un inconvénient avec ces dispositifs de l'art antérieur concerne la fiabilité du dispositif, notamment lors de l'actionnement. Ainsi, on prévoit généralement des ponts sécables entre deux parties mobiles l'une par rapport à l'autre lors de l'actionnement, notamment pour accumuler de l'énergie dans la main de l'utilisateur et définir de manière prédéterminée le seuil de force nécessaire pour réaliser l'actionnement. Ces ponts sécables présentent toutefois l'inconvénient de laisser parfois des résidus ou vestiges de pont sécable susceptibles de gêner lors de l'actionnement du dispositif.

Les documents WO9946055, WO0245866, WO2015001281, WO2017118827, US2016296957, WO2017191400, US6708846 et US6321942 décrivent des dispositifs de l'état de la technique.

La présente invention a pour but de fournir un dispositif de distribution nasale de poudre qui ne reproduit pas les inconvénients susmentionnés.

La présente invention a aussi pour but de fournir un dispositif de distribution nasale de poudre qui améliore la fiabilité du dispositif pendant l'actionnement.

La présente invention a également pour but de fournir un dispositif de distribution nasale de poudre qui est simple et peu coûteux à fabriquer et à assembler.

La présente invention a donc pour objet un dispositif de distribution nasale de poudre comportant un réservoir contenant au moins une dose de poudre, une tête de distribution nasale destinée à être insérée dans une narine d'un utilisateur, ladite tête de distribution nasale comportant un orifice de distribution, et une chasse d'air générant, lors de l'actionnement dudit dispositif de distribution nasale de poudre, un écoulement d'air comprimé pour distribuer une dose de poudre dans ladite narine à travers ledit orifice de distribution, ladite chasse d'air comprenant une chambre d'air, disposée dans une jupe, et un piston qui lors de l'actionnement du dispositif coulisse de manière étanche dans ladite chambre d'air pour comprimer l'air contenu dans ladite chambre d'air, ledit piston étant solidaire d'un organe d'actionnement, dans lequel, avant actionnement, au moins un pont sécable est prévu entre ladite jupe et ledit organe d'actionnement, chaque pont sécable étant formé sur ladite jupe et coopérant avec une projection radiale formée sur ledit organe d'actionnement, chaque projection radiale comportant une étendue axiale supérieure à celle du pont sécable respectif et formant une rampe axiale inclinée de part et d'autre du pont sécable respectif.

Avantageusement, ladite jupe comporte à proximité de son bord axialement inférieur une bride inférieure, radialement saillante vers l'intérieur, ledit au moins un pont sécable étant formé sur un bord radialement interne de ladite bride inférieure.

Avantageusement, la quantité de matière de chaque pont sécable est minimale au niveau du sommet de la projection radiale respective.

Avantageusement, ledit organe d'actionnement comporte sur sa surface latérale externe des profils saillants radialement vers l'extérieur, tels que des nervures longitudinales, chaque projection radiale s'étendant radialement vers l'extérieur au moins autant que lesdits profils saillants.

Avantageusement, ledit réservoir contient une seule dose de poudre, distribuée lors d'un seul actionnement dudit dispositif de distribution nasale de poudre.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique en section transversale d'un dispositif de distribution nasale de poudre selon un mode de réalisation avantageux, en position de repos, avant actionnement,
la figure 2 est une vue similaire à celle de la figure 1, en début d'actionnement,
les figures 3 à 5 sont des vues similaires à celle de la figure 1, montrant différentes positions en cours d'actionnement,
la figure 6 est une vue similaire à celle de la figure 1, en fin d'actionnement,
les figures 7 et 8 sont des vues de détail, respectivement de dessus et de côté, d'un pont sécable selon un mode de réalisation avantageux, et
la figure 9 est une vue schématique partielle en perspective des deux parties du dispositif reliées par des ponts sécables avant actionnement.

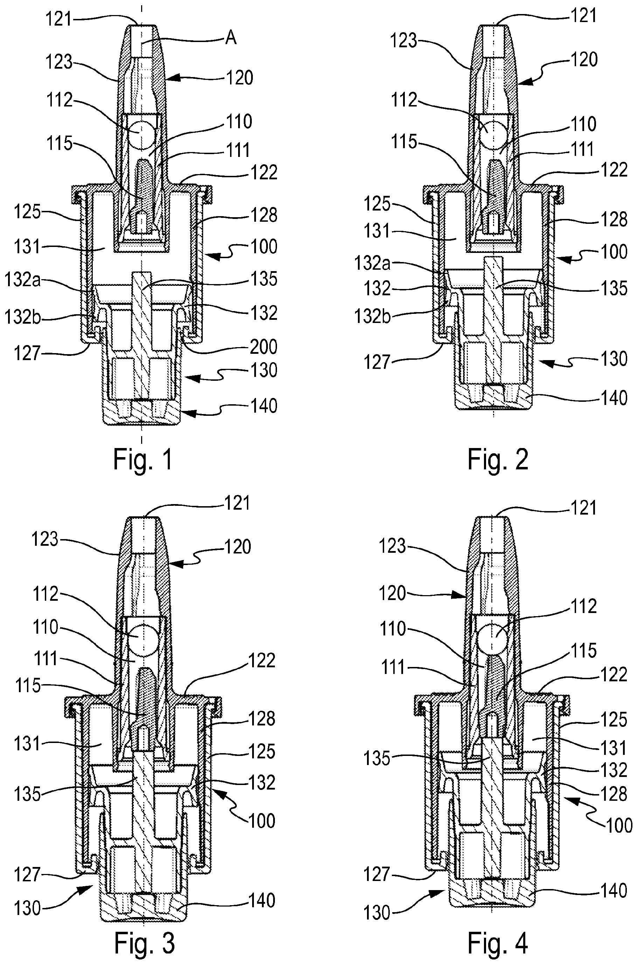

Dans la description, les termes "axial" et "radial" se réfèrent à l'axe longitudinale A du dispositif représenté sur la figures 1. Les termes "proximal" et "distal" se réfèrent à l'orifice de distribution dudit dispositif. Les termes "haut", "bas", "supérieur" et "inférieur" se réfèrent à la position droite du dispositif représentée sur les dessins.

L'invention s'applique plus particulièrement à des dispositifs du type unidose poudre tel que celui représenté sur les figures. Bien entendu, d'autres types de dispositifs de distribution nasale de poudre sont aussi envisageables.

Le dispositif 100 représenté sur les figures comporte un réservoir 110 contenant une seule dose de poudre. Des dispositifs avec un réservoir contenant plus d'une dose sont possibles. De même, des dispositifs comportant plusieurs réservoirs contenant chacune une seule dose sont aussi possibles.

Une tête de distribution nasale 120 est assemblée sur ledit réservoir 110, ladite tête étant destinée à être insérée dans une narine d'un utilisateur. Ladite tête de distribution nasale comporte un orifice de distribution 121. La tête de distribution 120 comporte avantageusement un repose-doigt 122 s'étendant radialement pour faciliter l'actionnement. Un manchon creux 123 s'étend axialement vers le haut à partir dudit repose-doigt 122 et se termine au niveau dudit orifice de distribution 121. De préférence, ce manchon creux 123 est de dimension radiale réduite pour pouvoir être inséré dans une narine au moment de l'actionnement. Du côté opposé du repose-doigt 122, une jupe 125 s'étend axialement vers le bas à partir dudit repose-doigt 122. Ladite jupe 125 comporte à proximité de son bord axialement inférieur une bride inférieure 127, radialement saillante vers l'intérieur.

Le dispositif 100 comporte en outre une chasse d'air 130 générant, lors de l'actionnement dudit dispositif 100, un écoulement d'air comprimé pour distribuer une dose de poudre dans ladite narine à travers ledit orifice de distribution 121. Ladite chasse d'air comprend une chambre d'air 131 et un piston 132 coulissant de manière étanche dans ladite chambre d'air 131 pour comprimer l'air contenu dans ladite chambre d'air 131 et ainsi générer ledit écoulement d'air comprimé. Le piston 132 comporte de préférence une lèvre supérieure 132a et une lèvre inférieure 132b. La chambre d'air 131 est formée par un cylindre axial creux 128 qui est solidaire, de préférence monobloc, avec le repose-doigt 122 de la tête de distribution 120. Le côté inférieur dudit cylindre creux 128 est ouvert et obturé par ledit piston 132. La jupe 125 est alors avantageusement disposée autour dudit cylindre creux 128, et peut notamment être formée par un manchon creux fixé, par exemple encliqueté, dans le repose-doigt 122 de la tête de distribution 120. La chambre d'air 131 est avantageusement ouverte sur l'atmosphère avant actionnement.

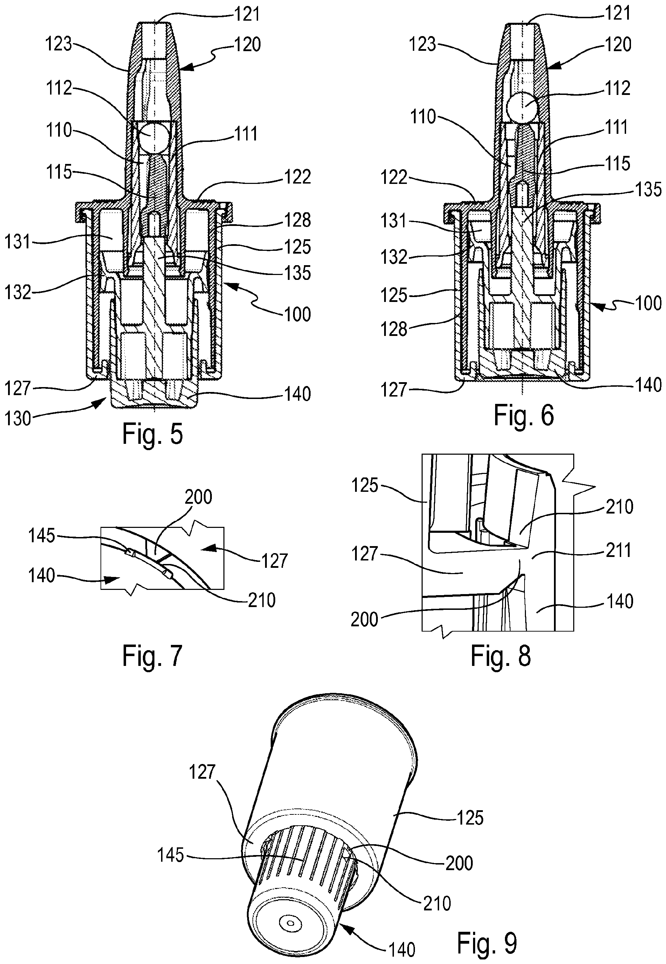

Le piston 132 est solidaire d'un organe d'actionnement 140 sur lequel l'utilisateur va appuyer lors de l'actionnement pour déplacer le piston 132 dans la chambre d'air 131. Cet organe d'actionnement 140 peut éventuellement comporter sur sa surface latérale externe des profils saillants, tels que des nervures longitudinales 145, visibles sur la figure 9.

Avant actionnement, au moins un pont sécable 200 est prévu entre la jupe 125 et l'organe d'actionnement 140. Avantageusement chaque pont sécable 200 est formé sur le bord radialement interne de la bride radiale 127 de la jupe 125 et s'étend jusqu'à l'organe d'actionnement 140. Le point de cassure, généralement défini par une quantité de matière réduite, est formé au niveau du contact avec l'organe d'actionnement 140. Ces ponts sécables 200 permettent d'empêcher un actionnement accidentel ou non souhaité, pendant le stockage ou le transport, ou lors d'une chute accidentelle. Ils nécessitent l'application d'une force prédéterminée pour être cassés, ce qui permet de générer une certaine précompression dans la main de l'utilisateur. Il peut y avoir un nombre quelconque de ponts sécables, par exemple trois répartis autour de la périphérie du dispositif.

Dans l'exemple représenté sur les figures, le réservoir 110 est formé par un tube creux 111 ouvert à ses deux extrémités axiales, et fermé à son extrémité proximale par un élément de fermeture 112, tel qu'une bille, et fermé à son extrémité distale par un insert 115. Cet insert 115 comporte une extension axiale formant tige, et peut, lors de l'actionnement, coulisser dans ledit tube creux 111 pour chasser ledit élément de fermeture 112 hors de sa position de fermeture. Dans cet exemple, le piston 132 de la chasse d'air 130 est solidaire d'une projection axiale 135 qui s'étend en direction proximale, et qui, lors de l'actionnement, va se déplacer ensemble avec le piston 132 lors de la compression de l'air contenu dans la chambre d'air 131. Lorsque ladite projection 135 du piston 132 vient en contact avec ledit insert 115 du réservoir 110, une continuation du déplacement du piston 132 va provoquer le coulissement dudit insert 115 dans ledit tube creux 111 hors de sa position de fermeture. Ledit insert 115 va d'une part ouvrir le passage entre la chasse d'air 130 et le réservoir 110 et d'autre part provoquer l'expulsion de l'élément de fermeture 112. Ainsi, l'air comprimé dans la chambre d'air 131 va s'écouler dans ledit réservoir et entrainer la dose de poudre hors dudit réservoir en direction dudit orifice de distribution 121. Les documents WO9946055, WO0245866, WO2015001281 et WO2017118827 décrivent des dispositifs de ce type. Bien entendu, d'autres types de dispositifs sont aussi possibles.

Selon l'invention, chaque pont sécable 200 coopère avec une projection radiale 210 formée sur l'organe d'actionnement.

Chaque projection radiale 210 comporte une étendue axiale supérieure à celle du pont sécable et forme une rampe axiale inclinée de part et d'autre du pont sécable 200, comme visible sur la figure 8.

La quantité de matière du pont sécable 200 est de préférence minimale au niveau de la partie la plus saillante ou sommet 211 de la projection radiale 210, pour garantir que le pont sécable se cassera à cet endroit.

L'invention permet d'éviter tout risque de frottements du pont sécable une fois cassé, lors de la course d'actionnement. En effet, dès que le pont sécable 200 est cassé, en tout début d'actionnement, l'organe d'actionnement 140 se déplace axialement par rapport à la jupe 125, de sorte que le pont sécable 200 se trouve décalé par rapport au sommet 211 de la projection radiale 210, et donc en éloignement de la surface externe de l'organe d'actionnement 140. On élimine ainsi les risques que le pont sécable vienne frotter contre cette surface externe lors de l'actionnement.

Ce risque est d'autant plus important lorsque la surface externe de l'organe d'actionnement 140 comporte des nervures 145. En effet, dans ce cas, si l'organe d'actionnement tourne légèrement après cassure des ponts sécables, ceux-ci peuvent se retrouver face à une nervure, et donc engendrer des frottements potentiellement gênants.

La présente invention prévoit que les projections radiales 210 s'étendent radialement au moins autant que lesdites nervures 145, pour limiter voire supprimer ce risque.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution nasale de poudre (100) comportant un réservoir (110) contenant au moins une dose de poudre, une tête de distribution nasale (120) destinée à être insérée dans une narine d'un utilisateur, ladite tête de distribution nasale comportant un orifice de distribution (121), et une chasse d'air (130) générant, lors de l'actionnement dudit dispositif de distribution nasale de poudre (100), un écoulement d'air comprimé pour distribuer une dose de poudre dans ladite narine à travers ledit orifice de distribution (121), ladite chasse d'air comprenant une chambre d'air (131), disposée dans une jupe (125), et un piston (132) qui lors de l'actionnement du dispositif coulisse de manière étanche dans ladite chambre d'air (131) pour comprimer l'air contenu dans ladite chambre d'air (131), ledit piston (132) étant solidaire d'un organe d'actionnement (140), **caractérisé en ce qu'**avant actionnement, au moins un pont sécable (200) est prévu entre ladite jupe (125) et ledit organe d'actionnement (140), chaque pont sécable (200) étant formé sur ladite jupe (125) et coopérant avec une projection radiale (210) formée sur ledit organe d'actionnement (140), chaque projection radiale (210) comportant une étendue axiale supérieure à celle du pont sécable (200) respectif et formant une rampe axiale inclinée de part et d'autre du pont sécable (200) respectif.

2. Dispositif selon la revendication 1, dans lequel ladite jupe (125) comporte à proximité de son bord axialement inférieur une bride inférieure (127), radialement saillante vers l'intérieur, ledit au moins un pont sécable (200) étant formé sur un bord radialement interne de ladite bride inférieure (127).

3. Dispositif selon la revendication 1 ou 2, dans lequel la quantité de matière de chaque pont sécable (200) est minimale au niveau du sommet (211) de la projection radiale (210) respective.

4. Dispositif selon l'une quelconque des revendications précédentes, dans lequel ledit organe d'actionnement (140) comporte sur sa surface latérale externe des profils saillants radialement vers l'extérieur, tels que des nervures longitudinales (145), chaque projection radiale (210) s'étendant radialement vers l'extérieur au moins autant que lesdits profils saillants (145).

5. Dispositif selon l'une quelconque des revendications précédentes, dans lequel ledit réservoir (110) contient une seule dose de poudre, distribuée lors d'un seul actionnement dudit dispositif de distribution nasale de poudre (100).

## Patentansprüche

1. Vorrichtung zur nasalen Verabreichung eines Pulvers (100) aufweisend einen Vorratsbehälter (110), der wenigstens eine Pulverdosis enthält, einen Kopf zur nasalen Verabreichung (120) zur Einführung in ein Nasenloch eines Benutzers, wobei der Kopf zur nasalen Verabreichung eine Abgabeöffnung (121) und eine Luftausstoßeinrichtung (130) aufweist, die bei der Betätigung der Vorrichtung zur nasalen Verabreichung eines Pulvers (100) einen Druckluftstrom erzeugt, um eine Pulverdosis durch die Abgabeöffnung (121) in das Nasenloch abzugeben, wobei die Luftausstoßeinrichtung eine Luftkammer (131), die in einem Mantel (125) angeordnet ist, und einen Kolben (132) umfasst, der bei der Betätigung der Vorrichtung dicht in der Luftkammer (131) gleitet, um die Luft zu komprimieren, die in der Luftkammer (131) enthalten ist, wobei der Kolben (132) mit einem Betätigungsglied (140) fest verbunden ist, **dadurch gekennzeichnet, dass** vor Betätigung wenigstens eine abtrennbare Brücke (200) zwischen dem Mantel (125) und dem Betätigungsglied (140) vorgesehen ist, wobei jede abtrennbare Brücke (200) auf dem Mantel (125) ausgebildet ist und mit einem Radialvorsprung (210) zusammenwirkt, der auf dem Betätigungsglied (140) ausgebildet ist, wobei jeder Radialvorsprung (210) eine axiale Erstreckung aufweist, die größer als jene der jeweiligen abtrennbaren Brücke (200) ist und eine geneigte axiale Rampe beiderseits der jeweiligen abtrennbaren Brücke (200) bildet.

2. Vorrichtung nach Anspruch 1, wobei der Mantel (125) in der Nähe seines axial unteren Randes einen unteren Flansch (127) aufweist, der radial nach innen hervorsteht, wobei die wenigstens eine abtrennbare Brücke (200) auf einem radial inneren Rand des unteren Flansches (127) ausgebildet ist.

3. Vorrichtung nach Anspruch 1 oder 2, wobei die Materialmenge jeder abtrennbaren Brücke (200) am Scheitelpunkt (211) des jeweiligen Radialvorsprungs (210) am kleinsten ist.

4. Vorrichtung nach einem der vorhergehenden Ansprüche, wobei das Betätigungsglied (140) auf seiner äußeren Seitenfläche Profile aufweist, die radial nach außen hervorstehen, wie etwa Längsrippen (145), wobei sich jeder Radialvorsprung (210) wenigstens so viel wie die hervorstehenden Profile (145) radial nach außen erstreckt.

5. Vorrichtung nach einem der vorhergehenden Ansprüche, wobei der Vorratsbehälter (110) eine einzelne Pulverdosis enthält, die bei einer einzelnen Betätigung der Vorrichtung zur nasalen Verabreichung eines Pulvers (100) abgegeben wird.

## Claims

1. Nasal powder delivery device (100) comprising a container (110) containing at least one dose of powder, a nasal delivery head (120) intended to be inserted in a nostril of a user, said nasal delivery head comprising a delivery aperture (121), and an air discharge system (130) generating, upon the actuation of said nasal powder delivery device (100), a flow of compressed air to deliver a dose of powder in said nostril through said delivery aperture (121), said air discharge system comprising an air chamber (131), arranged in a skirt (125), and a piston (132) which, upon actuation of the device, sealingly slides in said air chamber (131) to compress the air contained in said air chamber (131), said piston (132) being rigidly connected to an actuating member (140), **characterised in that** before actuation, at least one breakable bridge (200) is provided between said skirt (125) and said actuating member (140), each breakable bridge (200) being formed on said skirt (125) and cooperating with a radial projection (210) formed on said actuating member (140), each radial projection (210) comprising an axial extension greater than that of the respective breakable bridge (200) and forming an inclined axial ramp on either side of the respective breakable bridge (200).

2. Device according to claim 1, in which said skirt (125) comprises, in the vicinity of its axially lower edge, a lower flange (127), which radially projects inwards, said at least one breakable bridge (200) being formed on a radially inner edge of said lower flange (127).

3. Device according to claim 1 or 2, in which the quantity of material of each breakable bridge (200) is minimum at the top (211) of the respective radial projection (210).

4. Device according to any one of the preceding claims, in which said actuating member (140) comprises on its outer side surface profiles which radially project outwards, such as longitudinal ridges (145), each radial projection (210) extending radially outwards at least as much as said projecting profiles (145).

5. Device according to any one of the preceding claims, in which said container (110) contains one single dose of powder, distributed during one single actuation of said nasal powder delivery device (100).
